# EUROPÄISCHE PATENTANMELDUNG

(11) **EP 4 681 632 A1**
(43) Veröffentlichungstag der Anmeldung: **21.01.2026**
(21) Anmeldenummer: 25184303.3
(22) Anmeldetag: 20.06.2025
(51) Int. Cl.: A61B 5/12

(54) **VERFAHREN ZUM ERZEUGEN VON TESTSIGNALEN ZUM ERMITTELN EINER HBRBEEINTRACHTIGUNG EINER PERSON**

(30) Priorität: 21.06.2024 DE 102024117610
(71) Anmelder: Baumann UX GmbH, 85049 Ingolstadt (DE)
(72) Erfinder: Baumann, Fabian, 85049 Ingolstadt (DE)
(74) Vertreter: Leffers, Thomas

(57) **Zusammenfassung**

Die Erfindung betrifft ein Verfahren zum Erzeugen von Testsignalen zum Ermitteln einer Hörbeeinträchtigung einer Person, wobei das Verfahren folgende Schritte aufweist:
- Bereitstellen wenigstens eines ersten akustischen Signals, welches wenigstens ein Störgeräusch repräsentiert (2);
- Bereitstellen wenigstens eines zweiten akustischen Signals, welches Sprache repräsentiert (3);
- Für jedes des wenigstens einen ersten Signals, Zerlegen des entsprechenden Signals in wenigstens ein erstes Frequenzband und Erzeugen von Testsignalen zum Testen des Hörvermögens einer Person durch, für jedes des wenigstens einen ersten Frequenzbandes, jeweiliges Überlagern von wenigstens einem Teil des wenigstens einen zweiten akustischen Signals mit dem entsprechenden Frequenzband (4), und
- Ausgeben der erzeugten Testsignale (5).

## Beschreibung

Die Erfindung betrifft ein Verfahren zum Erzeugen von Testsignalen zum Ermitteln einer Hörbeeinträchtigung einer Person, basierend auf welchen eine Hörlösung auf die Bedürfnisse der jeweiligen Person beziehungsweise des jeweiligen Patienten optimal einstellbar ist.

Ein Hörgerät dient allgemein zur Ausgabe von Audiosignalen an einen gehörgeschädigten Patienten. Hierzu weist das Hörgerät für gewöhnlich zumindest einen Hörer auf, mittels welchem ein Audiosignal in ein Schallsignal umgewandelt wird. Das Hörgerät wird regelmäßig vom Nutzer im oder am Ohr getragen. Gewöhnliche Hörgeräte verändern dabei physikalische, akustische Signalgrößen so, dass ein mit dem Hörgerät ausgestatteter Patient verbessert hört. Die Anpassung des Hörgerätes erfolgt dabei durch Einstellung entsprechender Parameter beziehungsweise physikalischer Übertragungsgrößen, wie von frequenzabhängiger Verstärkung, Pegelbegrenzung etc., bis der Patient im Rahmen der dargebotenen Möglichkeiten befriedigt ist.

Die Einstellung dieser Parameter erfolgt dabei für gewöhnlich basierend auf Ergebnissen eines Hörtests. Dabei ist es bekannt, zunächst in kompletter Ruhe die Hörschwelle, das heißt die Lautstärke, welche pro Frequenz benötigt wird, damit der Patient einen Einzelton gerade noch so wahrnehmen kann, zu messen. Zudem wird dem Patienten eine Wortgruppe bei normaler Gesprächslaustärke, das heißt 65dB vorgespielt. Dabei kann die Wortgruppe zusätzlich zusammen mit einem 60dB Störlärm beziehungsweise einer Störgröße, das heißt Beitdband-Rauschen abgespielt werden beziehungsweise mit diesem überlagert werden.

Als nachteilig bei derartigen Verfahren erweist sich hierbei jedoch, dass hieraus keinerlei Aufschluss darüber gewonnen werden kann, welche Störgeräusche den individuellen Pateienten jeweils insbesondere betreffen und/oder wann, das heißt in welchen Situationen das Hörvermögen des individuellen Patienten während dessen Alltag insbesondere beeinträchtigt ist.

Aus der Druckschrift EP 0 674 464 A1 ist ein Hörgerät bekannt, bei welchem zur automatischen Umschaltung einem Verstärker- und Übertragungsteil ein Controller zugeordnet ist, der in Abhängigkeit von die jeweilige Umgebungssituation kennzeichnenden Eingangsgrößen eine Auswahl der im Datenträger gespeicherten Parametersätze oder von Parametern zur Veränderung von Übertragungscharakteristiken des Hörgerätes vornimmt.

Aus der Druckschrift DE 10 2007 054 152 A1 ist ein Verfahren zum Erzeugen von Testsignalen zum Testen des Hörvermögens einer Person bekannt.

Aus der Druckschrift US 2020/00000380 A1 ist ein Verfahren zum Testen eines Hörvermögens eines kleinen Kindes bekannt, wobei ein akustisches Signal in einzelne Frequenzbänder zerlegt wird, die einzelnen Frequenzbänder verstärkt und die verstärkten Frequenzbänder anschließend wieder zusammengefügt werden, so dass mehrere Frequenzbereiche gleichzeitig getestet werden können.

Der Erfindung liegt somit die Aufgabe zugrunde, ein Verfahren zum Erzeugen von Testsignalen zum Ermitteln einer Hörbeeinträchtigung einer Person anzugeben, basierend auf welchen eine Hörlösung auf die Bedürfnisse der jeweiligen Person beziehungsweise des jeweiligen Patienten optimal einstellbar ist.

Gelöst wird diese Aufgabe durch den Gegenstand der unabhängigen Ansprüche. Vorteilhafte Weiterbildungen sind Gegenstand der abhängigen Ansprüche.

Gemäß einer Ausführungsform der Erfindung wird diese Aufgabe gelöst durch ein Verfahren zum Erzeugen von Testsignalen zum Ermitteln einer Hörbeeinträchtigung einer Person, wobei das Verfahren ein Bereitstellen wenigstens eines ersten akustischen Signals, welches wenigstens ein Störgeräusch repräsentiert, ein Bereitstellen wenigstens eines zweiten akustischen Signals, welches Sprache beziehungsweise gesprochene Sprache repräsentiert, für jedes des wenigstens einen ersten Signals ein Zerlegen des entsprechenden Signals in wenigstens ein erstes Frequenzband und Erzeugen von Testsignalen zum Ermitteln einer Hörbeeinträchtigung einer Person durch, für jedes der wenigstens einen ersten Frequenzbandes, jeweiliges Überlagern von wenigstens einem Teil des wenigstens einen zweiten akustischen Signals mit dem entsprechenden Frequenzband, und ein Ausgeben der erzeugten Testsignale aufweist.

Unter einem Testsignal wird hierbei ein Signal, welches ausgegeben wird, um eine Hörbeeinträchtigung einer Person beziehungsweise eines Patienten zu testen, verstanden. Das Testsignal kann dabei beispielsweise aus einer natürlichen beziehungsweise gesprochenen Sprache, beispielsweise einzelnen Buchstaben oder einer Wortfolge, und einem Störgeräusch zusammengesetzt sein.

Unter einem akustischen Signal wird weiter ein Signal, welches über einen Ton beziehungsweise einen Lärm wiedergegeben wird, verstanden, beispielsweise eine gesprochene Sprache oder ein Störgeräusch.

Das die Signale in Frequenzbänder zerlegt werden, bedeutet weiter, dass diese in einzelne Komponenten, das heißt Frequenzbestandteile beziehungsweise Frequenzbereiche zerlegt, beziehungsweise aufgesplittet werden. Unter einem Frequenzbereich wird dabei ein begrenzter, zusammenhängender Abschnitt beziehungsweise Bereich dieser Frequenzbestandteile verstanden.

Dass die Signale anschließend überlagert werden, bedeutet zudem, dass diese aufsummiert beziehungsweise übereinandergelegt werden.

Durch das Verfahren werden somit Testsignale erzeugt, welche jeweils nur einzelne Bestandteile beziehungsweise Komponenten eines Störgeräusches aufweisen, wobei die Testsignale anschließend insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

Insgesamt wird somit ein Verfahren zum Erzeugen von Testsignalen zum Ermitteln einer Hörbeeinträchtigung einer Person angegeben, basierend auf welchen eine Hörlösung auf die Bedürfnisse der jeweiligen Person beziehungsweise des jeweiligen Patienten optimal einstellbar ist.

In einer Ausführungsform weist das Verfahren weiter einen Schritt eines, für jedes des wenigstens einen zweiten Signals, jeweiliges Zerlegen des entsprechenden Signals in wenigstens ein zweites Frequenzband auf, wobei die Testsignale zum Ermitteln einer Hörbeeinträchtigung einer Person durch jeweiliges Überlagern von jedem des wenigstens einen ersten Frequenzbandes mit einem oder mehreren des wenigstens einen zweiten Frequenzbandes erzeugt werden. Somit kann auch das Sprachsignal beziehungsweise die gesprochene Sprache in einzelne Komponenten, beispielsweise einzelne Buchstaben, zerlegt werden, so dass das Hörvermögen der entsprechenden Person noch genauer bestimmbar ist.

Das wenigstens eine erste Frequenzband und/oder das wenigstens eine zweite Frequenzband können dabei Frequenzbänder mit unterschiedlicher Breite aufweisen.

Unter Breite eines Frequenzbandes wird dabei der von diesem umfasste beziehungsweise abgedeckte Frequenzbereich verstanden.

Basierend auf Frequenzbändern mit unterschiedlicher Breite kann dabei ein entsprechender Hörtest noch verfeinert und/oder stufenweise erschwert werden, wodurch das Hörvermögen beziehungsweise die Hörbeeinträchtigung der entsprechenden Person noch genauer bestimmt werden kann, insbesondere welche Geräusche beziehungsweise Töne das Hörvermögen der entsprechenden Person tatsächlich beeinflussen.

Mit einer weiteren Ausführungsform der Erfindung wird auch ein Verfahren zum Ermitteln einer Hörbeeinträchtigung einer Person angegeben, wobei das Verfahren ein Erzeugen von Testsignalen zum Ermitteln einer Hörbeeinträchtigung der Person durch ein obenstehend beschriebenes Verfahren zum Erzeugen von Testsignalen zum Ermitteln einer Hörbeeinträchtigung einer Person und ein Ermitteln einer Hörbeeinträchtigung der Person basierend auf den erzeugten Testsignalen aufweist.

Somit wird ein Verfahren zum Ermitteln einer Hörbeeinträchtigung einer Person angegeben, welches auf Testsignalen, basierend auf welchen eine Hörlösung auf die Bedürfnisse der jeweiligen Person beziehungsweise des jeweiligen Patienten optimal einstellbar ist, basiert. Insbesondere basiert das Verfahren auf Testsignalen, welche jeweils nur einzelne Bestandteile beziehungsweise Komponenten eines Störgeräusches aufweisen, wobei die Testsignale insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

Dabei kann der Schritt des Ermittelns einer Hörbeeinträchtigung der Person basierend auf den erzeugten Testsignalen ein Simulieren von unterschiedlichen Ausgleichsarten für Hörverluste und ein Ermitteln der Hörbeeinträchtigung für wenigstens zwei der unterschiedlichen Ausgleichsarten aufweisen.

Unter Ausgleichsart für Hörverluste wird hierbei die Art, wie Störgeräusche verarbeitet werden können, beispielsweise mit Unterstützung eines Hörgerätes, beziehungsweise auf diese reagiert werden kann, verstanden. Dabei können beispielsweise Störgeräusche komplett herausgefiltert werden, oder können durch die Verarbeitung im Hörgerät erfasste Geräusche so wenig wie möglich verändert werden.

Hierdurch können neben der Ermittlung des optimalen Hörvermögens auch für den entsprechenden Patienten optimale Ausgleichsverfahren ermittelt werden, wodurch eine verbesserte Vorauswahl von für den entsprechenden Patienten geeigneten Hörlösungen beziehungsweise Hörgeräten ermöglicht wird.

Mit einer weiteren Ausführungsform der Erfindung wird weiter auch ein Verfahren zum Einstellen von Parametern eines Hörgerätes einer Person angegeben, wobei das Verfahren ein Ermitteln einer Hörbeeinträchtigung der Person durch ein obenstehend beschriebenes Verfahren zum Ermitteln einer Hörbeeinträchtigung einer Person und ein Einstellen von Parametern des Hörgerätes basierend auf der Hörbeeinträchtigung der Person aufweist.

Unter Parametern eines Hörgerätes werden hierbei Übertragungs- und/oder Verstärkungsfunktionen beziehungsweise -Größen des Hörgerätes verstanden.

Somit wird ein Verfahren zum Einstellen eines Hörgerätes angegeben, welches auf Testsignalen, basierend auf welchen eine Hörlösung auf die Bedürfnisse der jeweiligen Person beziehungsweise des jeweiligen Patienten optimal einstellbar ist, basiert. Insbesondere basiert das Verfahren auf Testsignalen, welche jeweils nur einzelne Bestandteile beziehungsweise Komponenten eines Störgeräusches aufweisen, wobei die Testsignale insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

Mit einer weiteren Ausführungsform der Erfindung wird zudem auch eine Vorrichtung zum Erzeugen von Testsignalen zum Ermitteln einer Hörbeeinträchtigung einer Person angegeben, wobei die Vorrichtung eine erste Bereitstellungseinheit, welche ausgebildet ist, wenigstens ein erstes akustisches Signal, welches wenigstens ein Störgeräusch repräsentiert, bereitzustellen, eine zweite Bereitstellungseinheit, welche ausgebildet ist, wenigstens ein zweites akustisches Signal, welches Sprache repräsentiert, bereitzustellen, eine Erzeugungseinheit, welche ausgebildet ist, für jedes des wenigstens einen ersten Signals das entsprechende Signal in wenigstens ein erstes Frequenzband zu zerlegen und Testsignale zum Testen des Hörvermögens einer Person durch, für jedes des wenigstens einen ersten Frequenzbandes, jeweiliges Überlagern von wenigstens einem Teil des wenigstens einen zweiten akustischen Signals mit dem entsprechenden Frequenzband zu erzeugen, und eine Ausgabeeinheit, welche ausgebildet ist, die erzeugten Testsignale auszugeben, aufweist.

Somit wird eine Vorrichtung zum Erzeugen von Testsignalen zum Ermitteln einer Hörbeeinträchtigung einer Person angegeben, basierend auf welchen eine Hörlösung auf die Bedürfnisse der jeweiligen Person beziehungsweise des jeweiligen Patienten optimal einstellbar ist. Durch die Vorrichtung werden insbesondere Testsignale erzeugt, welche jeweils nur einzelne Bestandteile beziehungsweise Komponenten eines Störgeräusches aufweisen, wobei die Testsignale anschließend insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

In einer Ausführungsform ist die Erzeugungseinheit weiter ausgebildet, für jedes des wenigstens einen zweiten Signals das entsprechende Signal in wenigstens ein zweites Frequenzband zu zerlegen und die Testsignale zum Ermitteln einer Hörbeeinträchtigung einer Person durch jeweiliges Überlagern von jedem des wenigstens einen ersten Frequenzbandes mit einem oder mehreren des wenigstens einen zweiten Frequenzbandes zu erzeugen. Somit kann auch das Sprachsignal beziehungsweise die gesprochene Sprache in einzelne Komponenten, beispielsweise einzelne Buchstaben, zerlegt werden, so dass das Hörvermögen der entsprechenden Person noch genauer bestimmbar ist.

Das wenigstens eine erste Frequenzband und/oder das wenigstens eine zweite Frequenzband können dabei Frequenzbänder mit unterschiedlicher Breite aufweisen. Basierend auf Frequenzbändern mit unterschiedlicher Breite kann dabei ein entsprechender Hörtest noch verfeinert werden, beispielsweise stufenweise erschwert werden, wodurch das Hörvermögen der entsprechenden Person noch genauer bestimmt werden kann, insbesondere welche Geräusche beziehungsweise Töne das Hörvermögen der entsprechenden Person tatsächlich beeinflussen.

Mit einer weiteren Ausführungsform der Erfindung wird außerdem auch ein System zum Ermitteln einer Hörbeeinträchtigung einer Person angegeben, wobei das System eine obenstehend beschriebene Vorrichtung zum Erzeugen von Testsignalen zum Ermitteln einer Hörbeeinträchtigung einer Person und eine Testeinheit, welche ausgebildet ist, die Hörbeeinträchtigung der Person basierend auf durch die Vorrichtung zum Erzeugen von Testsignalen zum Ermitteln der Hörbeeinträchtigung einer Person erzeugten Testsignalen zu testen, aufweist.

Somit wird ein System zum Ermitteln einer Hörbeeinträchtigung einer Person angegeben, welches auf Testsignalen, basierend auf welchen eine Hörlösung auf die Bedürfnisse der jeweiligen Person beziehungsweise des jeweiligen Patienten optimal einstellbar ist, basiert. Insbesondere basiert das System auf Testsignalen, welche jeweils nur einzelne Bestandteile beziehungsweise Komponenten eines Störgeräusches aufweisen, wobei die Testsignale insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

Dabei kann die Testeinheit ausgebildet sein, unterschiedliche Ausgleichsarten für Hörverluste zu simulieren und die Hörbeeinträchtigung für wenigstens zwei der unterschiedlichen Ausgleichsarten zu ermitteln. Hierdurch können neben der Ermittlung des optimalen Hörvermögens auch für den entsprechenden Patienten optimale Ausgleichsverfahren ermittelt werden, wodurch eine verbesserte Vorauswahl von für den entsprechenden Patienten geeigneten Hörlösungen beziehungsweise Hörgeräten ermöglicht wird.

Mit einer weiteren Ausführungsform der Erfindung wird außerdem auch ein System zum Einstellen von Parametern eines Hörgerätes einer Person angegeben, wobei das System ein obenstehend beschriebenes System zum Ermitteln einer Hörbeeinträchtigung einer Person und eine Einstelleinheit, welche ausgebildet ist, Parameter des Hörgerätes basierend auf der durch das System zum Ermitteln einer Hörbeeinträchtigung einer Person ermittelten Hörbeeinträchtigung der Person einzustellen, aufweist.

Somit wird ein System zum Einstellen eines Hörgerätes angegeben, welches auf Testsignalen, basierend auf welchen eine Hörlösung auf die Bedürfnisse der jeweiligen Person beziehungsweise des jeweiligen Patienten optimal einstellbar ist, basiert. Insbesondere basiert das System auf Testsignalen, welche jeweils nur einzelne Bestandteile beziehungsweise Komponenten eines Störgeräusches aufweisen, wobei die Testsignale insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

Mit einer weiteren Ausführungsform der Erfindung wird außerdem auch ein Computerprogramm mit Programmcode, um ein obenstehend beschriebenes Verfahren zum Erzeugen von Testsignalen zum Ermitteln einer Hörbeeinträchtigung einer Person auszuführen, wenn das Computerprogramm auf einem Computer ausgeführt wird, angegeben.

Das Computerprogramm hat dabei den Vorteil, dass diese ausgebildet sind, ein Verfahren zum Erzeugen von Testsignalen zum Ermitteln einer Hörbeeinträchtigung einer Person auszuführen, basierend auf welchen eine Hörlösung auf die Bedürfnisse der jeweiligen Person beziehungsweise des jeweiligen Patienten optimal einstellbar ist. Durch das Verfahren werden insbesondere Testsignale erzeugt, welche jeweils nur einzelne Bestandteile beziehungsweise Komponenten eines Störgeräusches aufweisen, wobei die Testsignale anschließend insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

Zusammenfassend ist festzustellen, dass mit der vorliegenden Erfindung ein Verfahren zum Erzeugen von Testsignalen zum Ermitteln einer Hörbeeinträchtigung einer Person angegeben wird, basierend auf welchen eine Hörlösung auf die Bedürfnisse der jeweiligen Person beziehungsweise des jeweiligen Patienten optimal einstellbar ist.

Die Erfindung wird nun anhand der beigefügten Figuren näher erläutert.

Es zeigen:
- Fig. 1.: zeigt ein Flussdiagramm eines Verfahrens zum Ermitteln einer Hörbeeinträchtigung einer Person gemäß Ausführungsformen der Erfindung;
- Fig. 2.: zeigt ein schematisches Blockschaltbild eines Systems zum Ermitteln einer Hörbeeinträchtigung einer Person gemäß Ausführungsformen der Erfindung.

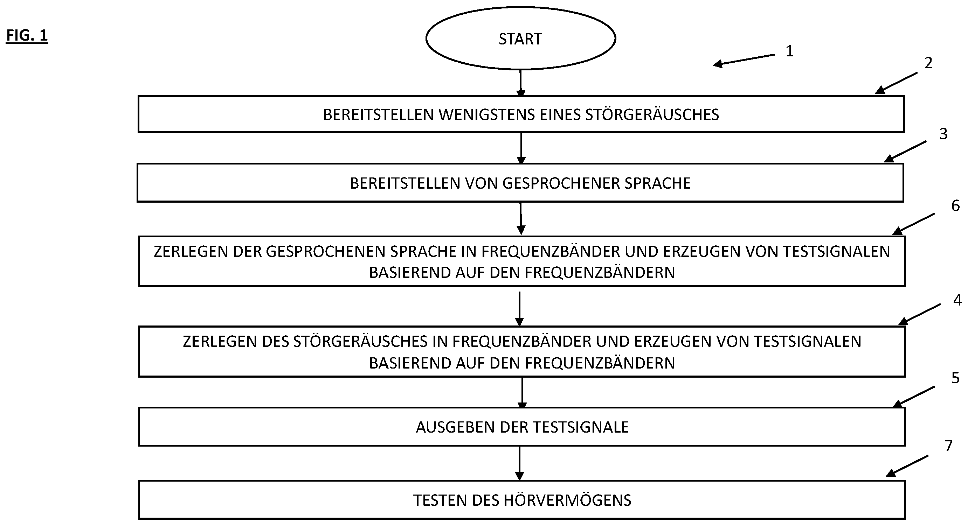

Fig. 1 zeigt ein Flussdiagramm eines Verfahrens zum Ermitteln einer Hörbeeinträchtigung einer Person 1 gemäß Ausführungsformen der Erfindung.

Für gewöhnlich ist der erste Berührungspunkt bei einer Hörgeräteversorgung der Hörtest. Dabei wird zunächst in kompletter Ruhe die Hörschwelle, also die Lautstärke, die pro Frequenz benötigt wird, damit ein Patient einen Einzelton gerade so wahrnehmen kann, gemessen. Zudem wird dem Patienten eine Wortgruppe in normaler Gesprächslautstärke, für gewöhnlich 65dB vorgespielt, welcher dieser anschließend nachsprechen soll. Kann der Patient dabei nicht mehr als 80% der Wortgruppe korrekt wiederholen, wird die Lautstärke der wiedergegebenen Sprachsignale für gewöhnlich so lange schrittweise, breitbandig, also ohne Frequenzanpassung erhöht, bis der Patient maximal gut versteht oder Unbehagen, welches durch die Unbehaglichkeitsschwelle repräsentiert wird, verspürt.

Dieser Messablauf berücksichtigt jedoch keinerlei Alltagssituationen. So treten die meisten Hörprobleme zuerst im Lärm beziehungsweise beim Auftreten von zusätzlichen Störgeräuschen auf, wodurch die mentale Anstrengung, die notwendig ist, um in einer gesellschaftlichen Situation, also in der Sprache und Störlärm gemeinsam auftreten, zurecht zu kommen, erhöht werden muss.

Wird die zuvor beschriebene Wortgruppe in 65dB mit zusätzlichem 60dB Störlärm wiedergegeben, können Einschränkung durch den Störlärm, beispielsweise Brandband-Rauschen verdeutlicht werden. Obwohl diese Messung bereits dem empfundenen Hörproblem im Lärm nahekommt, ist es nicht möglich eine direkte Verbesserung zu simulieren. Diese Messung, mit und ohne Störgeräusch, wird für gewöhnlich lediglich dazu verwendet, um die resultierende Verbesserung einer Hörlösung für die Krankenkassenbezuschussung abschließend zu beweisen.

Unabhängig vom verwendeten Testsignal oder der entsprechenden Zielsetzung, erfolgt auch die Anpassung des Hörgeräts selbst ohne die Präsenz von Störgeräuschen, da nur so die Verstärkungskurve des Hörsystems überlagerungsfrei erfasst werden kann. Ein zusätzliches Signal, welches den Störlärm einer realen Situation darstellt, wird jedoch aktuell nicht zur Feinanpassung des Hörsystems eingesetzt.

Jedes Hörgerät hat ferner eine vom Hersteller entwickelte dynamische Übertragungscharakteristik, die in einzelnen Merkmalen des Systems abgebildet wird, aber von außen nicht einsehbar ist. So sind Hörsysteme keine reinen Verstärker mehr, sondern basieren auf einem internen Algorithmus, welcher die Übertragung je nach erkannter Situation verändert. Dieses Verhalten ist vom anpassenden Hörakustiker nur bedingt änderbar oder vergleichbar. Anpassungsverfahren sind statisch und stellen in Ruhe das Hörvermögen des Patienten wieder her und nicht den maximal möglichen Nutzen und Komfort mit aktiven Merkmalen im Lärm.

Wie Fig. 1 zeigt, weist das Verfahren 1 einen Schritt 2 eines Bereitstellens wenigstens eines ersten akustischen Signals, welches wenigstens ein Störgeräusch repräsentiert, einen Schritt 3 eines Bereitstellens wenigstens eines zweiten akustischen Signals, welches eine gesprochene Sprache repräsentiert, für jedes des wenigstens einen ersten Signals einen Schritt 4 eines Zerlegens des entsprechenden Signals in wenigstens ein erstes Frequenzband und Erzeugens von Testsignalen zum Ermitteln einer Hörbeeinträchtigung einer Person durch, für jedes des wenigstens einen ersten Frequenzbandes, jeweiliges Überlagern von wenigstens einem Teil des wenigstens einen zweiten akustischen Signals mit dem entsprechenden Frequenzband, und einen Schritt 5 eines Ausgebens der erzeugten Testsignale aufweist.

Durch das Verfahren 1 werden Testsignale erzeugt, welche jeweils nur einzelne Bestandteile beziehungsweise Komponenten eines Störgeräusches aufweisen, wobei die Testsignale anschließend insbesondere dazu verwendet werden können, um herauszufinden, welche dieser Komponenten das Hörvermögen der entsprechenden Person tatsächlich beeinträchtigt. Basierend auf den erzeugten Testsignalen können insbesondere realistische Hörsituationen mit variablem Schwierigkeitsgrad, das heißt in ihren Einzelkomponenten steuerbare beziehungsweise zerlegbare Hörsituationen simuliert werden, wodurch Alltagsbeeinträchtigungen besser simuliert werden können.

Insgesamt wird somit ein Verfahren zum Erzeugen von Testsignalen zum Ermitteln einer Hörbeeinträchtigung einer Person 1 angegeben, basierend auf welchen eine Hörlösung auf die Bedürfnisse der jeweiligen Person beziehungsweise des jeweiligen Patienten optimal einstellbar ist.

Insbesondere können Testsignale erzeugt werden, basierend auf welchen bekannte Situationen, beispielsweise ein Restaurantbesuch dargeboten beziehungsweise wiedergegeben werden können. Basierend auf diesen Testsignalen können Höreinschränkungen, welche im Alltag des Patienten tatsächlich auftreten, erfasst werden, beziehungsweise ermittelt werden, wann tatsächlich Deutlichkeitsverluste auftreten.

Gemäß den Ausführungsformen der Fig. 1 weist das Verfahren 1 weiter einen Schritt 6 eines, für jedes des wenigstens einen zweiten Signals, Zerlegens des wenigstens eines zweiten Signals in wenigstens ein zweites Frequenzband auf, wobei die Testsignale zum Testen des Hörvermögens einer Person in Schritt 4 durch jeweiliges Überlagern von jedem des wenigstens einen ersten Frequenzbandes mit einem oder mehreren des wenigstens einen zweiten Frequenzbandes erzeugt werden.

Somit wird auch eine Spektralanalyse des Sprachsignals beziehungsweise des gesprochenen Signals durchgeführt.

Dabei kann beispielsweise jedes der ersten Frequenzbänder sukzessive mit jedem der zweiten Frequenzbänder überlagert werden.

Basierend auf entsprechenden Testsignalen kann dann beispielsweise überprüft werden, wie tief bei einem individuellen Patienten Störgeräusche in verschiedenen Bändern abgesenkt und wie hoch Sprache in einzelnen Bändern entsprechend erhöht werden sollte.

Gemäß den Ausführungsformen der Fig. 1 weisen das wenigstens eine erste Frequenzband und das wenigstens eine zweite Frequenzband jeweils wenigstens zwei Frequenzbänder mit unterschiedlicher Breite auf.

Die Breite kann dabei beispielsweise zwischen komplette Buchstabenfolgen umfassenden Frequenzbändern, Breiten von 1KHz beziehungsweise ein Logatom umfassenden Frequenzbändern und lediglich einen Buchstaben umfassenden Frequenzbändern variieren.

Basierend auf den unterschiedlichen Breiten können Änderungen der einzelnen Frequenzbänder in mehreren Stufen und Breiten wiedergegeben werden, wodurch beispielsweise ein stufenweiser Ausgleich bei der Einstellung von Parametern eines Hörgerätes simuliert werden kann. Beispielsweise kann dabei ein Buchstaben-Störgeräusch-Verhältnis während eines Hörtestes so lange angeglichen werden, bis der entsprechende Buchstabe verstanden wird.

Wie Fig. 1 zeigt, weist das Verfahren 1 zudem einen Schritt 7 eines Ermittelns einer Hörbeeinträchtigung einer Person beziehungsweise eines Patienten basierend auf den erzeugten Testsignalen auf.

Insbesondere kann dabei basierend auf den einzelnen Testsignalen der in entsprechenden Situationen benötigte Signal-Rausch-Abstand, beispielsweise der Signal-Rausch-Abstand pro Buchstabe ermittelt werden.

Dabei kann eine Kompensierung und eine Verbesserungspotenzialerfassung des frequenzabhängigen Hörvermögens in geräuschvollen Situationen, insbesondere in Alltagssituationen genau simuliert werden.

Durch das Verfahren 1 können dabei insbesondere auch Maskierungseffekte und ein schrittweiser Ausgleich eines Hörverlustes simuliert werden.

Dass Hörbeeinträchtigungen und nicht der physikalische Hörverlust ermittelt wird, hat zudem den Vorteil, dass das Alter beziehungsweise die altersabhängige Fähigkeit, Geräusche im Gehirn nachzuvollziehen beziehungsweise nachzubilden, berücksichtigt wird. So hat beispielsweise für gewöhnlich ein 30-jähriger weniger Einschränkungen bei gleichem Hörverlust als ein 70-jähriger mit eingeschränktem Hirntakt.

Die Ergebnisse des Hörtestes können dabei beispielsweise in Form einer Matrix festgehalten werden.

Gemäß den Ausführungsformen der Fig. 1 weist der Schritt 7 des Testens des Hörvermögens der Person basierend auf den erzeugten Testsignalen weiter ein Simulieren von unterschiedlichen Ausgleichsarten für Hörverluste und ein Ermitteln der Hörbeeinträchtigung für wenigstens zwei der unterschiedlichen Ausgleichsarten auf.

Insbesondere wird dabei simuliert, was das Hörgerät theoretisch leisten kann.

Beispielsweise kann dabei eine Windgeräuschunterdrückung und/oder eine unterschiedlich starke Unterdrückung von Störgeräuschen simuliert werden.

Basierend auf den Testergebnissen können anschließend beispielsweise Parameter von Hörgeräten eingestellt beziehungsweise approximiert werden.

Insbesondere unterstützen die gewonnen, genaueren Ergebnisse bei der Entscheidung für eine Hörhilfe und auch bei der Auswahl von optimalen Ausgleichsstrategien.

Dabei kann beispielsweise die Regeltiefe entsprechend individuell angepasst werden, das heißt wie stark das Gerät regeln beziehungsweise die Lautstärke entsprechend angepasst werden sollte.

Das Verfahren 1 ermöglicht somit die für den individuellen Patienten passende Kombination aus Verarbeitungsstrategie, Verstärkereinstellungen und technischer Ausstattung zu finden und umzusetzen.

Die Verfahrensschritte können dabei insbesondere vor der Anschaffung eines Hörgerätes durchgeführt werden. Zudem kann das Verfahren aber auch dynamisch, das heißt während des Tragens eines Hörgerätes durchgeführt werden, wobei basierend auf den entsprechenden Testergebnissen Parameter des Hörgerätes neu eingestellt beziehungsweise optimiert werden können.

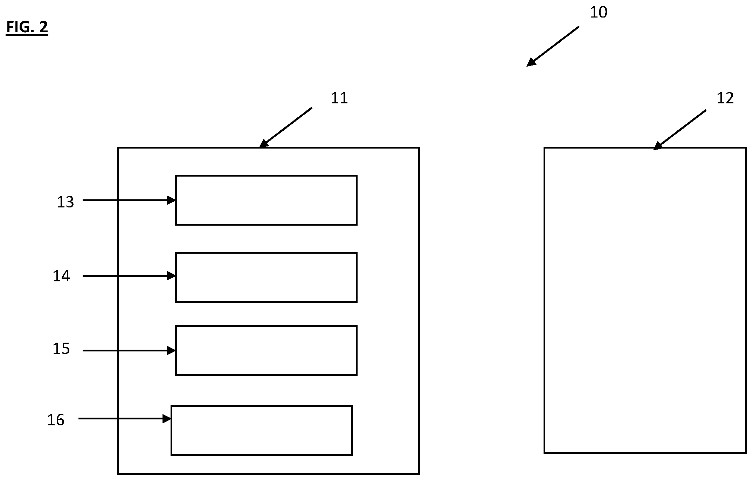

Fig. 2 zeigt ein schematisches Blockschaltbild eines Systems zum Ermitteln einer Hörbeeinträchtigung einer Person 10 gemäß Ausführungsformen der Erfindung.

Wie Fig. 2 zeigt, weist das dargestellte System 10 eine Vorrichtung zum Erzeugen von Testsignalen zum Ermitteln einer Hörbeeinträchtigung einer Person 11 und eine Testeinheit 12, welche ausgebildet ist, eine Hörbeeinträchtigung der Person basierend auf durch die Vorrichtung zum Erzeugen von Testsignalen zum Ermitteln einer Hörbeeinträchtigung einer Person erzeugten Testsignalen zu testen, auf.

Die Testeinheit kann dabei auf Audioausgabeeinheiten und entsprechenden Recheneinheiten basieren und ausgebildet sein, durch eine Ton- und Sprachaudiometrie die Hörbeeinträchtigung der Person in entsprechenden Frequenzbereichen zu testen.

Die dargestellte Vorrichtung 11 weist weiter eine erste Bereitstellungseinheit 12, welche ausgebildet ist, wenigstens ein erstes akustisches Signal, welches wenigstens ein Störgeräusch repräsentiert, bereitzustellen, eine zweite Bereitstellungseinheit 13, welche ausgebildet ist, wenigstens ein zweites akustisches Signal, welches Sprache repräsentiert, bereitzustellen, eine Erzeugungseinheit 14, welche ausgebildet ist, für jedes des wenigstens einen ersten Signals das entsprechenden Signal in wenigstens ein erstes Frequenzband zu zerlegen und Testsignale zum Ermitteln einer Hörbeeinträchtigung einer Person durch, für jedes des wenigstens einen ersten Frequenzbandes, jeweiliges Überlagern von wenigstens einem Teil des wenigstens einen zweiten akustischen Signals mit dem entsprechenden Frequenzband zu erzeugen, und eine Ausgabeeinheit 15, welche ausgebildet ist, die erzeugten Testsignale auszugeben, auf.

Bei der ersten und der zweiten Bereitstellungseinheit kann es sich dabei insbesondere um Empfänger handeln, welche ausgebildet sind, entsprechende Audiosignale zu empfangen, wobei insbesondere die erste und die zweite Bereitstellungseinheit jeweils auch ein Mikrofon umfassen können, um akustische Signale aufzunehmen.

Die Erzeugungseinheit kann ferner beispielsweise basierend auf einem in einem Speicher hinterlegten und durch einen Prozessor ausführbaren Code realisiert werden.

Die Ausgabeeinheit kann ferner insbesondere eine Audioausgabeeinheit wie ein Lautsprecher sein.

Gemäß den Ausführungsformen der Fig. 2 ist die Erzeugungseinheit 14 dabei weiter ausgebildet, für jedes des wenigstens einen zweiten Signals das entsprechende Signal in wenigstens ein zweites Frequenzband zu zerlegen und die Testsignale zum Testen des Hörvermögens einer Person durch jeweiliges sukzessives Überlagern von jedem des wenigstens einen ersten Frequenzbandes mit einem oder mehreren des wenigstens einen zweiten Frequenzbandes zu erzeugen.

Zudem weisen das wenigstens eine erste Frequenzband und das wenigstens eine zweite Frequenzband wiederum jeweils wenigstens zwei Frequenzbänder mit unterschiedlicher Breite auf.

Gemäß den Ausführungsformen der Fig. 2 ist die Testeinheit 12 zudem wiederum ausgebildet, unterschiedliche Ausgleichsarten für Hörverluste zu simulieren und die Hörbeeinträchtigung für wenigstens zwei der unterschiedlichen Ausgleichsarten zu ermitteln.

Das dargestellte System ist dabei zudem ausgebildet, ein obenstehend beschriebenes Verfahren zum Ermitteln einer Hörbeeinträchtigung einer Person auszuführen.

## Patentansprüche

1. Verfahren zum Erzeugen von Testsignalen zum Testen des Hörvermögens einer Person, wobei das Verfahren folgende Schritte aufweist:
- Bereitstellen wenigstens eines ersten akustischen Signals, welches wenigstens ein Störgeräusch repräsentiert (2);
- Bereitstellen wenigstens eines zweiten akustischen Signals, welches Sprache repräsentiert (3);
- Für jedes des wenigstens einen ersten Signals, Zerlegen des entsprechenden Signals in wenigstens ein erstes Frequenzband und Erzeugen von Testsignalen zum Ermitteln einer Hörbeeinträchtigung einer Person durch, für jedes des wenigstens einen ersten Frequenzbandes, jeweiliges Überlagern von wenigstens einem Teil des wenigstens einen zweiten akustischen Signals mit dem entsprechenden Frequenzband (4), wobei die Testsignale derart erzeugt werden, dass diese jeweils nur einzelne Bestandteile des wenigstens einen Störgeräusches aufweisen; und
- Ausgeben der erzeugten Testsignale (5).

2. Verfahren nach Anspruch 1, wobei das Verfahren weiter einen Schritt eines, für jedes des wenigstens einen zweiten Signals, Zerlegen des wenigstens einen zweiten Signals in wenigstens ein zweites Frequenzband (6) aufweist, und wobei die Testsignale zum Ermitteln einer Hörbeeinträchtigung einer Person durch jeweiliges Überlagern von jedem des wenigstens einen ersten Frequenzbandes mit einem oder mehreren des wenigstens einen zweiten Frequenzbandes erzeugt werden.

3. Verfahren nach Anspruch 1 oder 2, wobei das wenigstens eine erste Frequenzband und/oder das wenigstens eine zweite Frequenzband Frequenzbänder mit unterschiedlicher Breite aufweist.

4. Verfahren zum Ermitteln einer Hörbeeinträchtigung einer Person, wobei das Verfahren (1) folgende Schritte aufweist:
- Erzeugen von Testsignalen zum Testen einer Hörbeeinträchtigung der Person durch ein Verfahren zum Erzeugen von Testsignalen zum Testen einer Hörbeeinträchtigung einer Person nach einem der Ansprühe 1 bis 3; und
- Ermitteln einer Hörbeeinträchtigung der Person basierend auf den erzeugten Testsignalen (7).

5. Verfahren (1) nach Anspruch 4, wobei der Schritt des Ermittelns einer Hörbeeinträchtigung der Person basierend auf den erzeugten Testsignalen ein Simulieren von unterschiedlichen Ausgleichsarten für Hörverluste und ein Ermitteln einer Hörbeeinträchtigung für wenigstens zwei der unterschiedlichen Ausgleichsarten aufweist.

6. Verfahren zum Einstellen von Parametern eines Hörgerätes einer Person, wobei das Verfahren folgende Schritte aufweist:
- Ermitteln einer Hörbeeinträchtigung der Person durch ein Verfahren zum Ermitteln einer Hörbeeinträchtigung einer Person nach Anspruch 4 oder 5; und
- Einstellen von Parametern des Hörgerätes basierend auf der Hörbeeinträchtigung der Person.

7. Vorrichtung zum Erzeugen von Testsignalen zum Ermitteln einer Hörbeeinträchtigung einer Person, wobei die Vorrichtung (11) eine erste Bereitstellungseinheit (13), welche ausgebildet ist, wenigstens ein erstes akustisches Signal, welches wenigstens ein Störgeräusch repräsentiert, bereitzustellen, eine zweite Bereitstellungseinheit (14), welche ausgebildet ist, wenigstens ein zweites akustisches Signal, welches Sprache repräsentiert, bereitzustellen, eine Erzeugungseinheit (15), welche ausgebildet ist, für jedes des wenigstens einen ersten Signals das entsprechenden Signal in wenigstens ein erstes Frequenzband zu zerlegen und Testsignale zum Ermitteln einer Hörbeeinträchtigung einer Person durch, für jedes des wenigstens einen ersten Frequenzbandes, jeweiliges Überlagern von wenigstens einem Teil des wenigstens einen zweiten akustischen Signals mit dem entsprechenden Frequenzband zu erzeugen, wobei die Testsignale derart erzeugt werden, dass diese jeweils nur einzelne Bestandteile des wenigstens einen Störgeräusches aufweisen, und eine Ausgabeeinheit (16), welche ausgebildet ist, die erzeugten Testsignale auszugeben, aufweist.

8. Vorrichtung (11) nach Anspruch 7, wobei die Erzeugungseinheit (15) weiter ausgebildet ist, für jedes des wenigstens einen zweiten Signals das entsprechende Signal in wenigstens ein zweites Frequenzband zu zerlegen und die Testsignale zum Ermitteln einer Hörbeeinträchtigung einer Person durch jeweiliges Überlagern von jedem des wenigstens einen ersten Frequenzbandes mit einem oder mehreren des wenigstens einen zweiten Frequenzbandes zu erzeugen.

9. Vorrichtung (11) nach Anspruch 7 oder 8, wobei das wenigstens eine erste Frequenzband und/oder das wenigstens eine zweite Frequenzband Frequenzbänder mit unterschiedlicher Breite aufweist.

10. System zum Ermitteln einer Hörbeeinträchtigung einer Person, wobei das System (10) eine Vorrichtung zum Erzeugen von Testsignalen zum Ermitteln einer Hörbeeinträchtigung einer Person (11) nach einem der Ansprüche 7 bis 9 und eine Testeinheit (12), welche ausgebildet ist, die Hörbeeinträchtigung der Person basierend auf durch die Vorrichtung zum Erzeugen von Testsignalen zum Ermitteln einer Hörbeeinträchtigung einer Person (11) erzeugten Testsignalen zu ermitteln, aufweist.

11. System (10) nach Anspruch 10, wobei die Testeinheit ausgebildet ist, unterschiedliche Ausgleichsarten für Hörverluste zu simulieren und die Hörbeeinträchtigung für wenigstens zwei der unterschiedlichen Ausgleichsarten zu ermitteln.

12. System zum Einstellen von Parametern eines Hörgerätes einer Person, wobei das System ein System zum Ermitteln einer Hörbeeinträchtigung einer Person nach Anspruch 10 oder 11 und eine Einstelleinheit, welche ausgebildet ist, Parameter des Hörgerätes basierend auf der durch das System eine System zum Ermittelten einer Hörbeeinträchtigung einer Person ermittelten Hörbeeinträchtigung der Person einzustellen, aufweist.

13. Computerprogramm mit Programmcode, um ein Verfahren zum Erzeugen von Testsignalen zum Testen des Hörvermögens einer Person einem der Ansprüche 1 bis 3 auszuführen, wenn das Computerprogramm auf einem Computer ausgeführt wird.
